# EUROPÄISCHE PATENTSCHRIFT

(11) **EP 4 638 584 B1**
(45) Veröffentlichungstag und Bekanntmachung des Hinweises auf die Patenterteilung: **27.05.2026**
(21) Anmeldenummer: 22843144.1
(22) Anmeldetag: 19.12.2022
(51) Int. Cl.: C08K 3/36, C08K 5/5415, C08K 5/5419, C09D 183/04

(54) **SILICONHARZ-HOHLPARTIKEL UND VERFAHREN ZU DEREN HERSTELLUNG**
HOLLOW SILICONE RESIN PARTICLES AND METHOD FOR THE PRODUCTION THEREOF
PARTICULES CREUSES DE RÉSINE DE SILICONE ET LEUR PROCÉDÉ DE PRODUCTION

(43) Veröffentlichungstag der Anmeldung: 29.10.2025
(73) Patentinhaber: Wacker Chemie AG, 81671 München (DE)
(72) Erfinder: KNÖR, Sebastian, 81671 München (DE)
(74) Vertreter: Wimmer, Andreas
(86) Internationale Anmeldenummer: PCT/EP2022/086714
(87) Internationale Veröffentlichungsnummer: WO 2024/132096

(56) Entgegenhaltungen:
- US-A1- 2009 004 418
- US-B1- 6 180 236

## Beschreibung

Die Erfindung betrifft Hohlpartikel, aufgebaut aus einem hohlen Kern und einer Hülle aus kondensationsvernetztem Siliconharz und partikulärem Feststoff und ein Verfahren zu deren Herstellung.

Hohlkörperpartikel finden breite Anwendung beispielsweise als leichte Füllstoffe zur Reduktion der Dichte von polymeren oder keramischen Bauteilen, oder zur Absorption, Transport und Freisetzung von aktiven Inhaltsstoffen, wie Duftstoffe, Pflegestoffe oder Wirkstoffe, beispielsweise in kosmetischer oder medizinischer Anwendung oder in Haushaltsmittel oder Waschmittel.

Die Herstellung von Hohlkörperpartikeln ist sehr aufwändig und verwendet meist ein hartes oder weiches Templat, auf welchem die Hülle aufgebaut wird und das anschließend sehr aufwändig wieder entfernt wird. Diese Vorgehensweisen sind ökologisch und ökonomisch äußerst nachteilig.

WO2007113095 und WO2021121562 beschreiben Polysiloxan-basierte Kern-Hülle Partikel, welche sehr vorteilhafte Eigenschaften in kosmetischer Anwendung und als Additiv in zahlreichen technischen Anwendungen haben, wo sie insbesondere Vorteile durch Oberflächeneffekte haben. Aufgrund der gefüllten Struktur können diese Partikel aber keine funktionellen Stoffe im Inneren absorbieren.

US2009004418 beschreibt Siliconharz-Hohlpartikel mit einer Partikelgröße kleiner 1 mm, wobei die Hülle aus einer Siliconharz-Zusammensetzung aus SiO_{4/2} Einheiten, RSiO_{3/2} Einheiten, and R₂SiO_{2/2} Einheiten gebildet wird. Die Herstellung ist sehr aufwändig und ökologisch und ökonomisch äußerst nachteilig, da zunächst entweder ein Templatpartikel, beispielsweise ein organischer Polymerpartikel, und/oder ein toxisches organisches Lösemittel, beispielsweise Toluol oder Xylol, in Wasser dispergiert wird und anschließend mit reaktiven Silanen unter Ausbildung der Siliconharz-Hülle beschichtet wird, wobei der Kern in einem letzten Schritt mit einem organischen Lösemittel entfernt wird.

US 9,802,175 B2 beschreibt ein Verfahren zur Herstellung von Siliconharz-Hohlpartikel, aufgebaut aus RSiO_{3/2} Einheiten, mit einer Partikelgröße kleiner 200 nm. Die Herstellung ist sehr aufwändig und ökologisch und ökonomisch äußerst nachteilig, da zunächst ein organischer Templatpartikel, beispielsweise ein Polystyrol Partikel, ein Polyacrylat Partikel oder ein Polyvinylacetat Partikel hergestellt wird, welcher im zweiten Schritt mit Alkoxy-funktionellen Silanen unter Ausbildung der Siliconharz-Hülle beschichtet wird, wobei der Kern in einem dritten Schritt mit einem organischen Lösemittel und Hitze entfernt wird.

US 5945043 beschreibt ein Verfahren zur Herstellung von Polysiloxan-Hohlpartikeln, deren Hülle aus einem thermoplastischen Polysiloxan gebildet wird. Das thermoplastische Polysiloxan wird in einem Lösemittel gelöst und die Mischung in Wasser dispergiert. Die Dispersion wird mit einem Sprühtrockner versprüht, wobei Lösemittel und Wasser entfernt werden und sich die thermoplastischen Polysiloxan-Hohlpartikel formen. Die polymere Hülle der Partikel wird nicht vernetzt. Dadurch sind solche Partikel sensitive gegenüber Temperatur und Lösemittel. Die Herstellung ist wegen der Verwendung eines toxischen Lösemittels ökologisch und ökonomisch äußerst nachteilig.

WO14098107 beschreibt ein Verfahren, in dem Silica-Partikel als Templat für Herstellung von Polysiloxan-Hohlpartikel verwendet werden. Die Silica-Partikel werden dispergiert. Auf der Oberfläche der Templat-Partikel wird die Polysiloxan-Hülle durch Hydrolyse und Kondensation eines Alkoxy-funktionellen Silans oder Siloxans gebildet. Anschließend wird der Templat-Partikel herausgelöst und zersetzt. Das Verfahren ist ökologisch und ökonomisch äußerst nachteilig, da aufwändig herzustellende Silica Partikel als Templat verwendet werden, welche im letzten Verfahrensschritt herausgelöst und zersetzt werden.

Xue Wang et al. (Journal of Colloid and Interface Science 542 (2019) 144-150) beschreiben ein Verfahren zur Herstellung von Hohlpartikel über eine Pickering Emulsion. Dabei wird eine Lösung einer photopolymerisierbaren Verbindung in einem organischen Öl in einer wässrigen Phase emulgiert, wobei feinteilige Silicapartikel die Grenzphase stabilisieren. In einem zweiten Verfahrensschritt wird die photopolymerisierbare Verbindung an der Grenzphase polymerisiert und bildet gemeinsam mit den Silicapartikeln eine feste Hülle. Die Herstellung ist wegen der Verwendung eines organischen Öls als Templat ökologisch und ökonomisch äußerst nachteilig.

Alle genannten Verfahren haben als Nachteil, dass eine feste oder flüssige Templatverbindung für den Aufbau einer Kern-Hülle-Struktur verwendet wird, welche beschichtet wird, und im Anschluss aufwändig entfernt und entsorgt werden muss.

US6180236 B1 offenbart ein Verfahren zur Herstellung von Hohlpartikeln mit einer Hülle aus einer Siliconharz-Zusammensetzung, wobei die Partikel aus einer wässrigen Dispersion erhalten werden.

Gegenstand der Erfindung sind Hohlpartikel P, aufgebaut aus einem hohlen Kern K
und einer Hülle H, die eine Siliconharz-Zusammensetzung Z enthält, welche kondensationsvernetzte Siliconzusammensetzung X und partikulären Feststoff F enthält.

Der mittlere Partikeldurchmesser d50 der Hohlpartikel P liegt im Bereich 0,1 - 100 µm, vorzugsweise im Bereich 0,4 - 60 µm und bevorzugt im Bereich 0,8 - 40 µm.

Die Hohlpartikel P sind vorzugsweise im Wesentlichen sphärisch. Vorzugsweise ist die Sphärizität SPHT3 mindestens 0,8, bevorzugt mindestens 0,82, bestimmbar gemäß ISO 9276-6 mit einem Camsizer X2 von Retsch Technology.

Die Hohlpartikel P sind amphiphil, haben eine definierte und einheitliche Struktur und können in wässrigen und öligen Medien dispergiert werden.

Die Hohlpartikel P haben weiter den Vorteil, dass sie durch die geringe Dichte als Additiv in Formulierungen an die Oberfläche wandern und dadurch verstärkte Oberflächeneffekte zeigen. Außerdem eignen sie sich für die Anwendung als leichter Füllstoff, beispielsweise für Keramiken.

Die Hohlpartikel P können andere Substanze einlagern. Gefüllte Partikel können dies nicht.

Die Erfindung stellt auch ein einfaches und kostengünstiges Verfahren zur Herstellung des Hohlköperpartikel bereit, welches ohne Verwendung eines Templats auskommt.

Ein weiterer Gegenstand der Erfindung ist ein Verfahren zur Herstellung der Hohlpartikel P, bei dem in einem ersten Schritt eine Dispersion V enthaltend partikulären Feststoff F und Wasser mit kondensationsvernetzbarer Siliconzusammensetzung X1, welche bei 20°C flüssiges, alkoxygruppenhaltiges Siliconharz A und alkoxygruppenhaltiges Silan B enthält,
vermischt wird, wobei eine kontinuierliche Wasser enthaltende Phase und eine diskontinuierliche Phase enthaltend kondensationsvernetzbare Siliconzusammensetzung X1 entstehen,
und in einem zweiten Schritt die Siliconzusammensetzung X1 in der diskontinuierlichen Phase zur Siliconzusammensetzung X vernetzt wird, wobei die Hohlpartikel P entstehen.

Das vorteilhafte erfindungsgemäße Verfahren unterscheidet sich insbesondere dadurch von den Verfahren nach dem Stand der Technik, dass es ohne Verwendung eines flüssigen oder festen Templats auskommt. Die nach dem Stand der Technik verwendeten Template bilden einen Kern, auf dessen Oberfläche eine Hülle aufgebaut wird. Anschließend wird das Templat unter Bildung eines Hohlpartikels wieder entfernt.

Nach dem erfindungsgemäßen Verfahren bildet sich die Hülle H durch Kondensationsvernetzung der emulgierten kondensationsvernetzbaren Siliconzusammensetzung X1, an der Grenzphase zur Wasser-haltigen kontinuierlichen Phase der Emulsion E. Die emulgierten Tropfen der kondensationsvernetzbaren Siliconzusammensetzung X1 bilden also zunächst einen temporären Kern, auf dessen Oberfläche sich die kondensationsvernetzbare Siliconzusammensetzung X1 anschließend während der Vernetzung zur Siliconzusammensetzung X mit partikulären Feststoff F zu einer Hülle H unter Ausbildung des Hohlpartikel P verbindet. Nach dem erfindungsgemäßen Verfahren ist kein gesondertes, teures oder aufwändig herzustellendes Templat erforderlich, welches aufwändig abgetrennt, recycelt oder als Müll entsorgt werden muss. Dadurch ist das erfindungsgemäße Verfahren ökonomisch und ökologisch sehr vorteilhaft.

Vorzugsweise enthält die kondensationsvernetzbare Siliconzusammensetzung X1, jeweils bezogen auf die Gesamtmenge der Komponenten (A) und (B),
- (A) 50-90 Gew.% mindestens eines Siliconharzes A,
   aus Einheiten der Formeln (Ia), (Ib), (VII) und (Id)

      [R¹⁷SiO_{3/2}] (Ia)

      [SiO_{4/2}] (Ib)

      [R¹⁷₃SiO_{1/2}] (VII)

      [R¹⁷₂SiO_{2/2}] (Id)
   wobei
   R¹⁷ gleich oder unabhängig voneinander verschiedene einwertige, substituierte oder nicht substituierte organische Reste, die funktionelle Gruppen tragen oder nicht tragen, ein -OH oder eine Wasserstoffrest,
   bedeuten,
   mit den Maßgaben, dass
   - in (A) mindestens 20 mol% der Formel (Ia) oder (Ib) oder einer Mischung aus beiden enthalten sind,
   - in (A) höchstens 50 mol% der Formel (Ib) enthalten sind,
   - Alkoxygruppen als R¹⁷ zu mindestens 5 Gew.% in (A) enthalten sind, mit der Maßgabe, dass
   - (A) bei 20°C flüssig ist
- (B) 10-50 Gew.-% von mindestens ein Silan B der allgemeinen Formel

   R¹₍₄₋ₐ₎Si(OR)ₐ (II),

   in der
   - **R**: einen Kohlenwasserstoffrest mit 1 bis 16 Kohlenstoffatomen dessen Kohlenstoffkette durch nicht benachbarte Gruppen -O- unterbrochen sein kann,
   - **R¹**: gleiche oder unabhängig voneinander verschiedene einwertige Kohlenwasserstoffreste bedeutet, und
   - **a**: die Werte 2, 3 oder 4 bedeutet, wobei mindestens 20 Gew.-% der Silane B, bezogen auf die Gesamtmasse aller Silane B, das Merkmal a = 3 oder 4 erfüllen.

### Komponente (A)

Vorzugsweise enthält die erfindungsgemäß verwendete kondensationsvernetzbare Siliconzusammensetzung X1 55-85 Gew.-% eines oder mehrerer Siliconharze A, bevorzugt 60 bis 80 Gew.-%, jeweils bezogen auf die Gesamtmenge der Komponenten (A) und (B) .

Die Siliconharze A sind vorzugsweise solche, die ein Molekulargewicht Mw von mindestens 500, bevorzugt mindestens 600, besonders bevorzugt mindestens 700, und höchstens 5000, bevorzugt höchstens 4000, besonders bevorzugt höchstens 3000 haben, wobei die Polydispersität höchstens 20, bevorzugt höchstens 18, besonders bevorzugt höchstens 16, insbesondere höchstens 15 ist.

Die Siliconharze A enthalten mindestens 20 mol%, bevorzugt mindestens 30 mol%, besonders bevorzugt mindestens 40 mol%, insbesondere mindestens 50 mol% Wiederholungseinheiten der Formel (Ia) oder (Ib) oder einer Mischung der Formeln (Ia) und (Ib), wobei Wiederholungseinheiten der Formel (Ib) in einer Menge von höchstens 50 mol%, bevorzugt höchstens 40 mol%, besonders bevorzugt höchstens 20 mol% enthalten sind. In einer besonders bevorzugten Ausführungsform sind keine Einheiten (Ib) in den Siliconharzen A enthalten.

Wiederholungseinheiten der Formel (Id) können in einer Menge bis zu 80 mol%, vorzugsweise bis zu 70 mol%, besonders bevorzugt bis zu 60 mol%, insbesondere bis zu 50 mol% in den Siliconharzen A enthalten sein.

Die Siliconharze A enthalten Alkoxygruppen als R¹⁷ zu mindestens 5 Gew.%, bevorzugt mindestens 8 Gew.%, und insbesondere bevorzugt mindestens 10 Gew.%.

Beispiele für geeignete Alkoxygruppen als R¹⁷ sind Kohlenwasserstoffoxyreste mit 1 bis 16 C-Atomen die auch substituiert sein können. Besonders geeignet und somit bevorzugt sind Methoxy-, Ethoxy-, iso-Propoxy-, n-Butoxy- und tert.-Butoxy-reste und der p-Nitrophenoxyrest, insbesondere bevorzugt sind der Methoxy- und Ethoxyrest.

Alle weiteren R¹⁷ können unabhängig voneinander einwertige Kohlenwasserstoffreste sein, die substituiert oder unsubstituiert sind. Bevorzugt sind es reine Kohlenwasserstoffreste, bevorzugt mit 1 bis 16 Kohlenstoffatomen. Ausgewählte Beispiele für geeignete Kohlenwasserstoffreste Reste R¹⁷ sind Alkylreste, wie der Methyl-, Ethyl-, n-Propyl-, iso-Propyl-, n-Butyl-, isoButyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, und Octadecylreste, wie der n-Octadecylrest, Cycloalkylreste, wie Cyclopentyl-, Cyclohexyl-, Cycloheptyl- und Methylcyclohexylreste, Arylreste, wie der Phenyl-, Naphthyl-, Anthryl- und Phenanthrylrest, Alkarylreste, wie Tolylreste, Xylylreste und Ethylphenylreste, und Aralkylreste, wie der Benzylrest und der β-Phenylethylrest. Bevorzugte Kohlenwasserstoffreste als Reste R¹⁷ sind Methyl, n-Propyl-, iso-Propyl-, Phenyl-, n-Octyl-, oder iso-Octyl-Reste, der Methyl, der n-Propyl, der Phenyl- und der iso-Octyl-Rest besonders bevorzugt ist, und der Methyl- und der Phenyl-Rest insbesondere bevorzugt ist.

### Komponente (B)

Vorzugsweise enthält die kondensationsvernetzbare Siliconzusammensetzung X1 15-45 Gew.-% eines oder mehrerer Silane B, bevorzugt 20 bis 40 Gew.-%, jeweils bezogen auf die Gesamtmenge der Komponenten (A) und (B).

Vorzugsweise erfüllen mindestens 50 Gew.-%, bevorzugt mindestens 70% Gew.-%, besonders bevorzugt mindestens 90% Gew.-%, insbesondere bevorzugt mindestens 95 Gew.-% der Silane B, bezogen auf die Gesamtmasse aller Silane B, das Merkmal a = 3 oder 4, wobei in einer bevorzugten Ausführungsform mindestens 30 Gew.-%, bevorzugt mindestens 40% Gew.-%, besonders bevorzugt mindestens 50% Gew.-%, von mindestens einem Silane B mit a = 4 enthaltend ist, jeweils bezogen auf die Gesamtmasse aller Silane B.

Beispiele für geeignete Reste R sind Kohlenwasserstoffreste mit 1 bis 16 C-Atomen die auch substituiert sein können. Besonders geeignet und somit bevorzugt sind Methyl-, Ethyl-, iso-Propyl-, und tert.-Butylreste und der p-Nitrophenylrest, insbesondere bevorzugt sind der Methyl- und Ethylrest.

Die Reste R¹ können unabhängig voneinander einwertige Kohlenwasserstoffreste seine, die substituiert oder unsubstituiert sind. Bevorzugt sind es reine Kohlenwasserstoffreste, bevorzugt mit 1 bis 16 Kohlenstoffatomen. Ausgewählte Beispiele für geeignete Kohlenwasserstoffreste Reste R¹ sind Alkylreste, wie der Methyl-, Ethyl-, n-Propyl-, iso-Propyl-, n-Butyl-, isoButyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, und Octadecylreste, wie der n-Octadecylrest, Cycloalkylreste, wie Cyclopentyl-, Cyclohexyl-, Cycloheptyl- und Methylcyclohexylreste, Arylreste, wie der Phenyl-, Naphthyl-, Anthryl- und Phenanthrylrest, Alkarylreste, wie Tolylreste, Xylylreste und Ethylphenylreste, und Aralkylreste, wie der Benzylrest und der β-Phenylethylrest. Bevorzugte Kohlenwasserstoffreste als Reste R¹ sind Methyl, n-Propyl-, iso-Propyl-, Phenyl-, n-Octyl-, oder iso-Octyl-Reste, der Methyl, der n-Propyl, der Phenyl- und der iso-Octyl-Rest besonders bevorzugt ist, und der Methyl- und der Phenyl-Rest insbesondere bevorzugt ist.

Die kondensationsvernetzbare Siliconzusammensetzung X1 kann weitere feste oder flüssige Inhaltsstoffe I enthalten, mit der Maßgabe, dass die Kondensationsvernetzung der Siliconzusammensetzungen X1 und die Bildung der Hülle H nicht gestört werden. Beispiele für weitere Inhaltsstoffe I sind Katalysatoren, aktive und inaktive Füllstoffe, Inhibitoren, Hitzestabilisatoren, Lösungsmittel, Weichmacher, Farbpigmente, lösliche Farbstoffe, Sensibilisatoren, Photoinitiatoren, Haftvermittler, Leitfähigkeitsadditive, kosmetischen Stoffe, Duftstoffe, medizinischen oder kosmetischen Wirkstoffe

Fluoreszenzfarbstoffe, Fungizide, Duftstoffe, rheologische Additive, Korrosionsinhibitoren, Oxidationsinhibitoren, Lichtschutzmittel, Hitzestabilisatoren, flammabweisend machende Mittel, Mittel zur Beeinflussung der elektrischen Eigenschaften und Mittel zur Verbesserung der Wärmeleitfähigkeit. Diese Inhaltsstoffe können im Kern des Hohlpartikel P verbleiben und dadurch verkapselt, gespeichert, transportiert oder gezielt freigesetzt werden.

### Partikulärer Feststoff F

Bevorzugt handelt es sich bei dem erfindungsgemäß eingesetzten partikulären Feststoff F um bei 20°C und dem Druck der umgebenden Atmosphäre, also 1013 hPa, feste Partikel.

Der partikuläre Feststoff F weist bevorzugt eine Löslichkeit in Wasser bei pH 7,33 und einem Elektrolythintergrund von 0,11 Mol und einer Temperatur von 37°C von kleiner 0,1 g/l, besonders bevorzugt von kleiner als 0,05 g/l, auf, bei dem Druck der umgebenden Atmosphäre, also 1013 hPa.

Bevorzugt weist der partikuläre Feststoff F eine Molmasse größer 10 000 g/Mol, besonders bevorzugt eine Molmasse von 50 000 bis 50 000 000 g/Mol, insbesondere von 100 000 bis 10 000 000 g/Mol, auf, jeweils gemessen bevorzugt mittels statischer Lichtstreuung.

Bevorzugt weist der partikuläre Feststoff F eine spezifische BET-Oberfläche von 30 bis 500 m²/g, besonders bevorzugt 100 bis 300 m²/g, auf. Die BET-Oberfläche wird nach bekannten Verfahren gemessen, bevorzugt gemäß Deutscher Industrie Norm DIN 66131 und DIN 66132.

Bevorzugt weist der partikuläre Feststoff F eine Mohs'sche Härte größer als 1, besonders bevorzugt größer als 4, auf.

Bevorzugt handelt es sich bei dem eingesetzten partikulären Feststoff F um ein Metalloxid mit einem kovalenten Bindungs-Anteil in der Metall-Sauerstoff-Bindung, wie z.B. feste Oxide der Haupt- und Nebengruppenelemente, wie der 3. Hauptgruppe, wie Bor-, Aluminium-, Gallium- und Indiumoxid, der 4. Hauptgruppe wie Siliciumdioxid, Germaniumdioxid, und Zinnoxid sowie -dioxid, Bleioxid und -dioxid, oder ein Oxid der Nebengruppenelemente, wie Titandioxid, Zirkondioxid, Hafniumdioxid, Ceroxid oder Eisenoxid.

Bevorzugt handelt es sich bei den erfindungsgemäß eingesetzten Metalloxiden um Aluminium(III)-, Titan(IV)- und Silicium(IV)oxide, wie nasschemisch hergestellte, beispielsweise gefällte Kieselsäuren oder Kieselgele, oder in Prozessen bei erhöhter Temperatur hergestellte Aluminiumoxide, Titandioxide oder Siliciumdioxide, wie zum Beispiel pyrogen hergestellte Aluminiumoxide, Titandioxide oder Kieselsäuren.

Dabei ist die mittlere Partikelgröße des partikulären Feststoffs F oder gegebenenfalls Aggregate der Partikel bevorzugt kleiner als der mittlere Durchmesser d₅₀ der nach dem erfindungsgemäßen Verfahren gebildeten Emulsionströpfchen ohne die feinteiligen Partikel.

Die mittlere Partikelgröße des partikulären Feststoffs F ist kleiner als 1000 nm, bevorzugt zwischen 10 nm und 800 nm, besonders bevorzugt zwischen 50 nm und 500 nm und ganz besonders bevorzugt zwischen 75 nm und 300 nm, jeweils gemessen als mittleren hydrodynamischen Äquivalentdurchmesser mittels Photonenkorrelationsspektroskopie in 173° Rückstreuung mit einem Nanosizer ZS der Fa. Malver.

Die Methanolzahl des partikulären Feststoffs F ist vorzugsweise kleiner als 70, bevorzugt kleiner als 50, besonders bevorzugt kleiner als 40 und insbesondere bevorzugt kleiner als 30.

Zur Bestimmung der Methanolzahl werden definierte Mischungen von Wasser mit Methanol hergestellt, und dann die Oberflächenspannungen dieser Gemische mit bekannten Methoden bestimmt. In einem getrennten Experiment werden diese Wasser-Methanol-Mischungen mit definierten Mengen an Partikeln überschichtet und unter definierten Bedingungen geschüttelt (beispielweise schwaches Schütteln mit der Hand oder mit einem Taumelmischer für ca. 1 Minute). Bestimmt wird das Wasser-Alkohol-Gemisch, bei dem die Partikel eben noch nicht einsinken und das Wasser-Alkohol-Gemisch mit höherem Alkoholgehalt, bei dem die Partikel eben einsinken. Die Oberflächenspannung des letzteren Alkohol-Wasser-Gemisches liefert die kritische Oberflächenenergie γ_{crit} als Maß für die Oberflächenenergie γ der Partikel. Der Methanolgehalt in Wasser ergibt die Methanolzahl.

Der Kohlenstoffgehalt des partikulären Feststoffs F ist größer als 0 Gew.%, bevorzugt 0,1 - 4 Gew.%, besonders bevorzugt 0,25 - 3,5 Gew.% und ganz besonders bevorzugt 0,5 - 3 Gew.%, gemessen mittels Elementaranalyse an den trockenen partikulären Feststoffen.

In einer bevorzugten Ausführungsform handelt es sich beim partikulären Feststoff F um eine Kieselsäure S.

Kieselsäure S sind vorzugsweise teilweise mit Wasser benetzbare pyrogene und gefällte Kieselsäuren oder Gemische davon, die eine spezifische BET-Oberfläche von 30 bis 500 m²/g, besonders bevorzugt 100 bis 300 m²/g, aufweisen, wobei pyrogene Kieselsäure besonders bevorzugt sind. Die BET-Oberfläche wird nach bekannten Verfahren gemessen, bevorzugt gemäß Deutscher Industrie Norm DIN 66131 und DIN 66132.

Vorzugsweise ist Kieselsäure S mit einem geeigneten Hydrophobiermittel oberflächenbehandelt und dadurch hydrophob. Die Hydrophobierung ist so auszuführen, dass die Kieselsäure S noch teilweise mit Wasser benetzbar ist. Dies bedeutet erfindungsgemäß, dass die Methanolzahl der Kieselsäure S kleiner als 70, bevorzugt kleiner als 50, besonders bevorzugt kleiner als 40 und insbesondere bevorzugt kleiner als 30 ist. Bevorzugte Kieselsäure S weisen infolge einer Oberflächenbehandlung einen Kohlenstoffgehalt von mindestens 0,2 bis maximal 1,5 Gew.%, bevorzugt zwischen 0,4 und 1,4 Gew.%, besonders bevorzugt zwischen 0,6 bis 1,3 Gew.% auf. Beispielsweise handelt es sich bei den hydrophoben Gruppen um Si-gebundene Methyl oder Vinylgruppen. Methoden zur Hydrophobierung von Kieselsäuren sind dem Fachmann bekannt.

Bevorzugt als Kieselsäure S sind silanisierte pyrogene Kieselsäuren mit einer Methanolzahl von kleiner als 70, bevorzugt kleiner als 50, besonders bevorzugt kleiner als 40 und insbesondere bevorzugt kleiner als 30.

Ganz besonders bevorzugt sind teilweise mit Wasser benetzbare Kieselsäuren wie beschrieben in EP 1433749 A1 und DE 10349082 A1.

### Hohler Kern K

Die isolierten und getrockneten Hohlpartikel P einhalten einen hohlen Kern K. Dabei ist das durchschnittliche Verhältnis des mittleren Durchmesser des hohlen Kern K zum mittleren Durchmesser des Hohlpartikel P vorzugsweise größer 0,2, bevorzugt größer 0,3, jeweils ermittelt als Durchschnittswert von mindestens 5 Einzelpartikeln mittels elektronenmikroskopischen Aufnahmen wie TEM oder REM. Der hohle Kern K kann aus einem einzigen oder mehreren voneinander getrennten Hohlräumen bestehen.

Der hohle Kern K ist geeignet, um darin weitere Inhaltsstoffe I einzulagern.

### Hülle H

Die Hülle H enthält eine Siliconharz-Zusammensetzung Z aus der kondensationsvernetzten Siliconzusammensetzung X und dem partikulären Feststoff F. Die Hülle H bildet sich durch Kondensationsvernetzung der emulgierten kondensationsvernetzbaren Siliconzusammensetzung X1 an der Grenzphase zur Wasser-haltigen kontinuierlichen Phase der Emulsion. Die Grenzphase wird durch den partikulären Feststoff F stabilisiert, welcher während der Kondensationsvernetzung physikalisch und/oder chemisch in die sich bildende Hülle H eingebunden wird.

Die Hülle H hat vorzugsweise einen mittleren Durchmesser von mindestens 50 nm, bevorzugt mindestens 70 nm, jeweils ermittelt als Durchschnittswert von mindestens 5 Einzelpartikeln mittels elektronenmikroskopischen Aufnahmen wie TEM oder REM.

### Katalysator K

Bei weniger reaktiven kondensationsvernetzbaren Siliconzusammensetzungen X1 sind Katalysatoren K erforderlich, die gegebenenfalls die Hydrolyse und die Kondensation der Siliconharze A und Silane B bewirken. Diese Katalysatoren sind dem Fachmann bekannt. Es können sowohl Säuren als auch Basen sein oder auch Metallkatalysatoren, wie Gruppe IV Übergangsmetallkatalysatoren, Zinnkatalysatoren, wie sie üblicherweise zur Beschleunigung von Hydrolysen, Kondensationsreaktionen oder Umesterungsreaktionen verwendet werden. Als Säuren oder Basen kommen neben den bekannten Mineralsäuren und Metallsalzen auch saure oder basische Silane oder Siloxane in Frage.

Bevorzugte basische Katalysatoren sind NaOH, KOH, Ammoniak und NEt₃. Bei Verwendung von basische Katalysatoren K liegt der pH-Wert der Reaktionsmischung vorzugsweise im Bereich von pH 8 bis pH 12.

Bevorzugte saure Katalysatoren K sind p-Toluolsulfonsäure, wässrige oder gasförmige HCl, Schwefelsäure. Bei Verwendung von sauren Katalysatoren liegt der pH Wert der Reaktionsmischung vorzugsweise im Bereich von pH 1 bis pH 5.

### Hohlpartikel P

Siliconharz-Hohlpartikel nach dem Stand der Technik haben eine Hülle bestehend aus Siliconharz, welche durch Beschichtung und anschließendes Entfernen eines flüssigen oder festen Templats erhalten wird. Die Hülle aus Siliconharz ist hydrophob und nicht geeignet die Verwendung in hydrophilen Formulierungen und Produkten, insbesondere nicht geeignet für die Verwendung als Additiv in wässrigen Formulierungen.

Die erfindungsgemäßen Hohlpartikel P besitzen eine amphiphile Hülle H aus Siliconharz-Zusammensetzung Z aus der kondensationsvernetzten Siliconzusammensetzung X und teilweise Wasserbenetzbaren Feststoff F. Die erfindungsgemäßen Hohlpartikel P weisen bevorzugt eine Methanolzahl kleiner als 80, bevorzugt kleiner als 60, besonders bevorzugt kleiner als 50 und insbesondere bevorzugt kleiner als 40 auf. Dadurch können die erfindungsgemäßen Hohlpartikel P sowohl in hydrophilen als auch in hydrophoben Formulierungen und Produkten leicht verarbeitet werden.

Die erfindungsgemäßen Hohlpartikel P weisen vorzugsweise eine BET von größer 4 m²/g, vorzugsweise größer 10 m²/g, bevorzugt größer 20 m²/g auf.

Die erfindungsgemäßen Hohlpartikel P weisen vorzugsweise eine Schüttdichte von kleiner 0,28 g/cm³, vorzugsweise kleiner 0,25 g/cm³ auf.

### Verfahren zur Herstellung der Hohlpartikel P

Vorzugsweise enthält die kontinuierliche Phase mindestens 80 Gew.-%, insbesondere mindestens 90 Gew.-% Wasser. Vorzugsweise bildet sich ein Dreiphasengemisch, bei dem eine Emulsion von in Wasser schwer löslicher und mit Wasser nicht mischbarer kondensationsvernetzbarer Siliconzusammensetzung X1, die mittels teilhydrophobierter Kieselsäuren in der Wasserphase stabilisiert sind (Pickering Emulsionen). Die kondensationsvernetzbare Siliconzusammensetzung X1 wird in einem zur Herstellung der Partikel P geeigneten Verfahren nach dem Emulgieren vernetzt. Gegebenenfalls muss die kondensationsvernetzbare Siliconzusammensetzung X1 hydrolysiert werden, wenn es sich dabei beispielsweise um alkoxy- oder acetoxysubstituierte Silane oder Siloxane handelt. Bei ausreichend reaktiven Siliconzusammensetzungen X1, bewirkt dabei bereits das vorhandene Wasser gegebenenfalls die Hydrolyse und nachfolgend die Kondensation. Dabei ist das Verfahren so auszuführen, dass während der Emulgierung keine wesentliche Vernetzung stattfindet, da sonst keine feinteilige Emulsion gebildet wird. Bei weniger reaktiven kondensationsvernetzbaren Siliconzusammensetzungen X1 sind Katalysatoren K erforderlich, die gegebenenfalls die Hydrolyse und die Kondensation der Siloxane und Silane bewirken.

Das Verfahren ist im zweiten Schritt so auszuführen, dass die kondensationsvernetzbare Siliconzusammensetzung X1, welche die diskontinuierliche Phase bildet, an der Grenzfläche zur kontinuierlichen Wasser enthaltende Phase unter Kondensationsvernetzung reagiert und die Hülle H der Hohlpartikel P bildet und sich dabei mit dem die Grenzphase stabilisierenden partikulären Feststoff F physikalisch und/oder chemisch verbindet.

Dem Fachmann ist bewusst, dass die neu gebildeten, dispergierten Hohlpartikel P, bevor sie zum ersten Mal getrocknet wurden, mit flüssigem Spaltprodukt der Kondensationsvernetzung und/oder der kontinuierlichen Wasser enthaltende Phase gefüllt sind.

Die Größe der Hohlpartikel P lässt sich zum Beispiel durch die Emulgiertechnik, also etwa durch Größen wie die eingetragene Scherenergie, den Volumenanteil der Siliconzusammensetzung X1, die Menge von partikulären Feststoff F, den pH-Wert der kontinuierlichen Wasserphase und deren Ionenstärke, die Viskosität, die Reihenfolge der Dosierung, die Dosiergeschwindigkeit, oder durch die Reaktionsführung steuern, das heißt zum Beispiel durch die Reaktionstemperatur, die Reaktionszeit, die Konzentrationen der eingesetzten Rohstoffe. Die Auswahl und die Menge des gegebenenfalls eingesetzten Hydrolyse- und Kondensationskatalysators hat ebenfalls einen Einfluss auf die Partikelgröße.

Sofern weitere optionale feste oder flüssige Inhaltsstoffe I enthalten sind, werden diese vorzugsweise in einem ersten Schritt mit der kondensationsvernetzbaren Siliconzusammensetzung X1 unter Bildung der Mischung B homogen vermischt, bevor in einem weiteren Schritt mit Dispersion V emulgiert und anschließend unter Ausbildung des Hohlpartikels P vernetzt wird. Dadurch wird sichergestellt, dass sich alle optionalen festen oder flüssigen Inhaltsstoffe I nach dem Emulgieren im Inneren der Tröpfchen und nach der Vernetzung im Inneren des Hohlpartikels P befinden.

Bevorzugt sind die Pickering Emulsionen E der Mischung B im Wesentlichen frei von herkömmlichen, bei Raumtemperatur und dem Druck der umgebenden Atmosphäre nicht-partikulären flüssigen und festen, organischen oberflächenaktiven Substanzen, wie nichtionischen, kationischen und anionischen Emulgatoren ("organische Emulgatoren").

Organische Emulgatoren heißt hier keine Partikel und Kolloide, sondern Moleküle und Polymere, folgend der Definition von Molekülen, Polymeren, Kolloide und Partikel wie gegeben in "Dispersionen und Emulsionen", G. Lagaly, O. Schulz, R. Zindel, Steinkopff, Darmstadt 1997, ISBN 3-7985-1087-3, S.1-4.

Im Allgemeinen weisen diese organischen Emulgatoren eine Größe kleiner 1 nm, eine Molmasse < 10 000 g/Mol, einen Kohlenstoffgehalt > 50 Gew.-%, bestimmbar durch Elementaranalyse, sowie eine Mohs'sche Härte kleiner als 1 auf.

Zugleich weisen die organischen Emulgatoren, von denen die erfindungsgemäßen Emulsionen im Wesentlichen frei sind, zumeist eine Löslichkeit in Wasser bei 20°C und dem Druck der umgebenden Atmosphäre, also 900 bis 1100 hPa, homogen oder in Micellen-Form von größer 1 Gew.-% auf.

Die Pickering Emulsionen E der Mischung B können solche organischen Emulgatoren bis zu einer maximalen Konzentration von kleiner als 0,1 mal, bevorzugt kleiner als 0,01 mal, besonders bevorzugt kleiner als 0,001 mal, insbesondere kleiner als 0,0001 mal der kritischen Micellkonzentration dieser organischen Emulgatoren in der Wasserphase enthalten; dies entspricht einer Konzentration dieser organischen Emulgatoren, bezogen auf das Gesamtgewicht der erfindungsgemäßen Dispersion von kleiner 10 Gew.-%, bevorzugt kleiner 2 Gew.-%, besonders bevorzugt kleiner 1 Gew.-%, insbesondere 0 Gew.-%.

Zur Herstellung der partikelstabilisierten Pickering Emulsion E im ersten Schritt können sämtliche dem Fachmann bekannten Methoden zur Herstellung von Emulsionen eingesetzt werden. Es zeigte sich jedoch, dass sich besonders gut geeignete Emulsionen zur Erzeugung der Hohlpartikel P gemäß der folgenden Verfahren erhalten lassen:
Verfahren 1:
   - Vorlegen einer hochkonzentrierten Dispersion V, wobei das vorgelegte Volumen so bemessen ist, dass es die Gesamtmenge an benötigtem Feststoff F und nur eine Teilmenge an Wasser enthält.
   - Langsames Zudosieren des Gesamtvolumens an Mischung B unter ständigem Homogenisieren z.B. mittels eines schnelllaufenden Rührers, schnelllaufenden Dissolvers oder eines Rotor-Stator-Systems.
   - Anschließend langsames Zudosieren des gewünschten Restvolumens an Wasser gegebenenfalls unter ständigem Homogenisieren z.B. mittels eines schnelllaufenden Rührers, schnelllaufenden Dissolvers oder eines Rotor-Stator-Systems.
Verfahren 2:
   - Vorlegen der Dispersion V, wobei das vorgelegte Volumen so bemessen ist, dass es die Gesamtmenge an benötigtem Feststoff F und Wasser enthält.
   - Langsames Zudosieren des Gesamtvolumens an Mischung B unter ständigem Homogenisieren z.B. mittels eines - schnelllaufenden Rührers, schnelllaufenden Dissolvers, eines Rotor-Stator-Systems oder mittels Kapillaremulgators.
Verfahren 3:
   - Vorlegen des Gesamtvolumens an Mischung B.
   - Langsames Zudosieren einer hochkonzentrierten Dispersion V, unter ständigem Homogenisieren z.B. mittels eines schnelllaufenden Rührers, schnelllaufenden Dissolvers oder eines Rotor-Stator-Systems, wobei das zudosierte Volumen so bemessen ist, dass es die Gesamtmenge an benötigtem Feststoff F und nur eine Teilmenge an Wasser enthält.
   - Anschließend langsames Zudosieren des gewünschten Restvolumens an Wasser gegebenenfalls unter ständigem Homogenisieren z.B. mittels eines schnelllaufenden Rührers, schnelllaufenden Dissolvers oder eines Rotor-Stator-Systems
Verfahren 4:
   - Vorlegen des Gesamtvolumens an Mischung B.
   - Langsames Zudosieren der Dispersion V unter ständigem Homogenisieren z.B. mittels eines schnelllaufenden Rührers, schnelllaufenden Dissolvers oder eines Rotor-Stator-Systems, wobei das zudosierte Volumen so bemessen ist, dass es die Gesamtmenge an benötigtem Feststoff F und Wasser enthält.
Verfahren 5:
   - Vorlegen des Gesamtvolumens an Mischung B und der Dispersion V, wobei das vorgelegte Volumen so bemessen ist, dass es die Gesamtmenge an benötigtem Feststoff F und Wasser enthält.
   - Gemeinsames Homogenisieren z.B. mittels eines schnelllaufenden Rührers, schnelllaufenden Dissolvers oder eines Rotor-Stator-Systems.
Verfahren 6:
   - Vorlegen des Gesamtvolumens an Mischung B und einer hochkonzentrierten Dispersion V, wobei das vorgelegte Volumen so bemessen ist, dass es die Gesamtmenge an benötigtem Feststoff F und eine Teilmenge an Wasser enthält.
   - Gemeinsames Homogenisieren z.B. mittels eines schnelllaufenden Rührers, schnelllaufenden Dissolvers oder eines Rotor-Stator-Systems.
   - Anschließend langsames Zudosieren des gewünschten Restvolumens an Wasser gegebenenfalls unter ständigem Homogenisieren z.B. mittels eines schnelllaufenden Rührers, schnelllaufenden Dissolvers oder eines Rotor-Stator-Systems.

Bevorzugt sind Verfahren 1, 2, 5 und 6, wobei Verfahren 2 und 5 besonders bevorzugt sind.

Das Homogenisieren erfolgt vorzugsweise in mindestens einem Verfahrensschritt für mindestens 30 Sekunden, bevorzugt mindestens 1 Minute.

Die Herstellung der Dispersion V des partikulären Feststoffs F in Wasser, die in der erfindungsgemäßen Emulsion die homogene Phase bildet, kann grundsätzlich gemäß den bekannten Verfahren zur Herstellung von Partikeldispersionen erfolgen, wie das Einarbeiten mittels Rührorganen mit hoher Scherwirkung wie schnelllaufende Rührer, schnelllaufende Dissolver, Rotor-Stator-Systeme, Ultraschalldispergatoren oder Kugel- bzw. Perlmühlen.

Die Konzentration des partikulären Feststoffs F in der Dispersion V beträgt dabei zwischen 1 und 80 Gew.%, bevorzugt zwischen 10 und 60 Gew.%, besonders bevorzugt zwischen 10 und 40 Gew.% und ganz besonders bevorzugt zwischen 12 und 30 Gew.%.

In einem optionalen Verfahrensschritt wird die Pickering Emulsion E mit Wasser gegebenenfalls unter ständigem Homogenisieren z.B. mittels eines schnelllaufenden Rührers, schnelllaufenden Dissolvers oder eines Rotor-Stator-Systems verdünnt.

Die beschriebenen Verfahren können sowohl in kontinuierlicher als auch in diskontinuierlicher Form durchgeführt werden.

Die Temperatur im ersten Schritt der Emulgierung liegt zwischen 0 °C und 80 °C, bevorzugt zwischen 10 °C und 50 °C.

Der Emulgierprozess kann bei Normaldruck, also bei 900 bis 1100 hPa, bei erhöhtem Druck oder im Vakuum durchgeführt werden. Bevorzugt ist der Prozess bei Normaldruck durchgeführt.

Die Konzentration des partikulären Feststoffs F im Dreiphasengemisch aus Dispersion V und Mischung B des ersten Schritts beträgt dabei zwischen 1 und 80 Gew.%, bevorzugt zwischen 2 und 50 Gew.%, besonders bevorzugt zwischen 3 und 30 Gew.% und ganz besonders bevorzugt zwischen 4 und 20 Gew.%.

Die Konzentration der kondensationsvernetzbaren Siliconzusammensetzung X1 im Dreiphasengemisch aus Dispersion V und Mischung B des ersten Schritts beträgt dabei zwischen 1 und 80 Gew.%, bevorzugt zwischen 20 und 76 Gew.%, besonders bevorzugt zwischen 40 und 72 Gew.% und ganz besonders bevorzugt zwischen 50 und 70 Gew.%.

Die Konzentration Wasser im Dreiphasengemisch aus Dispersion V und Mischung B des ersten Schritts beträgt dabei zwischen 5 und 80 Gew.%, bevorzugt zwischen 10 und 70 Gew.%, besonders bevorzugt zwischen 15 und 60 Gew.% und ganz besonders bevorzugt zwischen 20 und 40 Gew.%.

Ausgehend vom dem oben beschriebenen Dreiphasengemisch lassen sich die Hohlpartikel P in einem zweiten Schritt nach folgenden Verfahren erhalten:
Das Dreiphasengemisch wird vorzugsweise durch Zugabe von Wasser auf einen Massenanteil von Wasser von 50 Gew.-% bis 90 Gew.-%, bevorzugt von 60 Gew.-% bis 80 Gew.-% verdünnt.

Das Dreiphasengemisch wird im zweiten Schritt vorzugsweise unter geringer Scherwirkung beispielsweise mittels eines langsam laufenden Dissolvers, Rotor-Stators oder Balkenrührers bis zur vollständigen inneren Vernetzung der Hohlpartikel P gerührt oder mittels geeigneter Aggregate geschüttelt.

Die Dauer des zweiten Verfahrensschritts ist vorzugsweise kürzer als 120 h, bevorzugt liegt sie zwischen 0 h bis 48 h, besonders bevorzugt 0,1 h bis 24 h und in einer speziellen Ausführung 0,25 h bis 12 h.

Gegebenenfalls können dem Dreiphasengemisch aus Dispersion V und Mischung B des ersten Schritts die Vernetzung beschleunigende und vervollständigende Katalysatoren K wie oben genannt zugesetzt werden. Die Zugabe kann dabei vor der Herstellung des Dreiphasengemischs direkt in die diskontinuierliche Phase oder kontinuierliche Phase, während der Emulgierung oder nachträglich in das fertige Dreiphasengemisch erfolgen.

Die Einsatzmenge der gegebenenfalls zugesetzten Katalysatoren liegt dabei in dem für Katalysatoren typischen Mengenbereich.

Die Reaktionstemperatur im zweiten Schritt liegt zwischen 0 °C und 100 °C, bevorzugt zwischen 10 °C und 90 °C und besonders bevorzugt zwischen 20 °C und 80 °C.

Gegebenenfalls kann die Reaktion unter einer InertgasAtmosphäre wie Stickstoff, Argon oder Kohlendioxid durchgeführt werden. Der Sauerstoffanteil ist dann kleiner 15 Vol.%, bevorzugt kleiner 10 Vol.% und besonders bevorzugt kleiner 5 Vol.%.

Gegebenenfalls können dem Dreiphasengemisch wasserlösliche organische Lösungsmittel wie Alkohole wie Methanol, Ethanol oder i-Propanol oder Ketone wie Aceton oder MEK oder Ether wie THF oder andere zugesetzt werden. Diese können im ersten Schritt oder vor oder während des zweiten Schritts zugesetzt werden.

Gegebenenfalls können dem Dreiphasengemisch Dispergierhilfsmittel, Schutzkolloide und / oder Tenside zugesetzt werden. Diese können im ersten Schritt oder vor oder während des zweiten Schritts zugesetzt werden.

Bevorzugt enthält das Dreiphasengemisch weniger als 5 Gew.-%, besonders bevorzugt weniger als 1 Gew.-%, insbesondere weniger als 0,1 Gew.-%, Dispergierhilfsmittel, Schutzkolloide und Tenside. In einer speziellen Ausführung ist das Dreiphasengemisch frei von Dispergierhilfsmitteln, Schutzkolloiden und Tensiden.

Gegebenenfalls enthält das Dreiphasengemisch anorganische oder organische Elektrolyte. Diese können entweder nach dem ersten Schritt, während des zweiten Schritts oder nach Abschluss des zweiten Schritts zugesetzt werden.

Die Ionenstärke des Dreiphasengemischs liegt dabei zwischen 0,01 mmol/l und 1 mol/l, bevorzugt zwischen 0,1 mmol/l und 500 mmol/l und besonders bevorzugt zwischen 0,5 mmol/l und 100 mmol/l.

Gegebenenfalls kann die Oberfläche der Hohlpartikel P durch Behandlung mit reaktiven Silanen oder Siloxanen modifiziert werden. Diese können entweder unmittelbar nach Abschluss der Herstellung der Pickering-Emulsion im ersten Schritt, während der Reaktionsphase oder nach Abschluss der Reaktionsphase im zweiten Schritt zugesetzt werden, vor der Isolierung der Hohlpartikel P oder nach der Isolierung der Partikel in flüssiger oder fester Phase. Die Behandlung ist dabei so durchzuführen, dass eine kovalente, chemische Anbindung des Silans oder Siloxans an den Partikeln erfolgt. Entsprechende Methoden und Verfahren sind dem Fachmann bekannt.

Der Feststoffanteil der Hohlpartikel P im Dreiphasengemisch bestehend aus der Summe der eingesetzten Feststoffe und dem Polymerisationsprodukt des polyadditionsfähigen, polykondensationsfähigen oder polymerisierbaren Materials liegt zwischen 5 Gew.-% und 70 Gew.-%, bevorzugt zwischen 10 Gew.-% und 50 Gew.-% und besonders bevorzugt zwischen 20 Gew.-% und 40 Gew.-%.

Gegebenenfalls kann das Dreiphasengemisch nach dem zweiten Schritt weiterhin unter Rühren gelagert werden. Dies kann beispielsweise mittels Balken- oder Ankerrührer erfolgen.

In einer bevorzugten Ausführungsform werden die Hohlpartikel P isoliert, vorzugsweise durch Sedimentation, Abfiltrieren oder Zentrifugieren, bevorzugt durch Filtration oder Zentrifugieren, besonders bevorzugt durch Zentrifugation.

Nach dem Isolieren werden die Hohlpartikel P vorzugsweise mit einer Waschflüssigkeit gewaschen, die vorzugsweise ausgewählt wird aus VE-Wasser, Methanol, Ethanol und Gemischen davon.

In einer bevorzugten Ausführungsform werden die Hohlpartikel P in Pulverform von der wässrigen Phase isoliert. Dies kann beispielsweise mittels Filtration, Sedimentation, Zentrifugieren oder durch Entfernen der flüchtigen Bestandteile durch Trocknen in Öfen oder Trocknern oder durch Sprühtrocknung oder durch Anlegen eines entsprechenden Vakuums erfolgen.

Durch Sprühtrocknen lässt sich ohne weitere Bearbeitung eine sehr hohe Feinheit der Partikel P erreichen. Statisch getrocknete Hohlpartikel P neigen zur Ausbildung loser Agglomerate, die durch geeignete Mahlverfahren, zum Beispiel Kugelmühle oder Luftstrahlmühle desagglomeriert werden können.

Die wässrige Dispersion der gehärteten Hohlpartikel kann für alle Zwecke eingesetzt werden, für die auch bisher wässrige Dispersionen verwendet werden. Die wässrige Dispersion kann eingesetzt werden für kosmetische und pharmazeutische Anwendungen, Putz- und Reinigungsmittel oder Anwendungen zur Veränderung der Grenzflächeneigenschaften von festen und flüssigen Substraten, wie z.B. Hydrophobiermittel, Haftvermittlern, Trennmitteln, Papierbeschichtungen oder Schaumkontrollmitteln, zur Herstellung von w/o/w- bzw. o/w/o-Mehrfachemulsionen, beispielsweise als Controlled-Release-Systeme oder zur Segregation von Reaktivstoffen.

Die gehärteten Hohlpartikel P werden insbesondere in kosmetischen und medizinischen Produkten und als leichter Füllstoff im Kunststoff- und Keramikbereich verwendet.

Die Hohlpartikel P zeigen ein sehr vorteilhaftes Verhalten, insbesondere für kosmetische Anwendungen. Sie neigen nicht zur Agglomeration oder zum Verblocken und sind dadurch außerordentlich leicht zu verteilen und bewirken ein samtiges Hautgefühl.

Im Vergleich zu nicht-erfinderischen, gefüllten Partikeln, die keinen hohlen Kern haben, weisen die Hohlpartikel P eine niedrigere Dichte auf oder können im Kern mit anderen funktionellen Stoffen gefüllt werden, beispielsweise mit aktiven Inhaltsstoffen, wie Duftstoffe, Pflegestoffe, Vitamine, UV Absorber oder Wirkstoffe und können diese transportieren und kontrolliert freisetzen.

Im Vergleich zu nicht-erfinderischen Hohlpartikeln, die keine amphiphile Hülle H aus Siliconharz-Zusammensetzung Z haben, können Silica-beschichtet Hohlpartikel eine größere Menge funktioneller Stoffe auf der Silica-Oberfläche absorbieren, beispielsweise Duftstoffe, Pflegestoffe, Vitamine oder UV-Absorber, oder medizinische Wirkstoffe und können diese transportieren und kontrolliert freisetzen.

Im Vergleich zu nicht-erfinderischen Partikeln, die keine amphiphile Hülle H aus Siliconharz-Zusammensetzung Z haben, verhalten sich die Silica-beschichtet Partikel amphiphil, d.h. sie sind sowohl in öligen als auch in wässrigen Flüssigkeiten gut dispergierbar.

Im Vergleich zu nicht-erfinderischen Partikeln, die keine amphiphile Hülle H aus Siliconharz-Zusammensetzung Z haben, ist die Oberfläche der erfindungsgemäßen Hohlpartikel besser durch Flüssigkeiten benetzbar. Dadurch können sie wesentlich einfacher und schneller in Flüssigkeiten, wie beispielsweise kosmetische Formulierungen, dispergiert werden und sie absorbieren auch deutlich schneller und einfacher Flüssigkeiten auf der Oberfläche, beispielsweise absorbieren sie Sebum bei kosmetischer Applikation auf der Haut.

### Messmethoden

- Molekulargewichtsverteilungen:
   Molekulargewichtsverteilungen werden als Gewichtsmittel Mw und als Zahlenmittel Mn bestimmt, wobei die Methode der Gelpermeationschromatographie (GPC bzw. Size Exclusion Chromatographie (SEC)) Anwendung findet mit Polystyrol Standard und Brechungsindexdetektor (RI-Detektor). Wo nicht anders ausgewiesen wird THF als Eluent verwendet und DIN 55672-1 angewendet. Die Polydispersität ist der Quotient Mw/Mn.
- Feststoffgehalt: 10 g wässrige Dispersion werden in einer Porzellanschale mit der gleichen Menge Ethanol versetzt und in einem N₂-gespülten Trockenschrank bei 150 °C zur Gewichtskonstanz eingedampft. Die Masse m_{S} des trockenen Rückstandes ergibt den Feststoffgehalt gemäß Feststoffgehalt / % = mₛ * 100 / 10 g.
- mittlerer Partikeldurchmesser (d50-Wert) und Partikeldurchmesser:
   Die Bestimmung des d₅₀-Werts erfolgte mit einem Camsizer X2 von Retsch Technology (Messprinzip: Dynamische Bildanalyse nach ISO 13322-2, Messbereich: 0,8 µm - 8 mm, Art der Analyse: Trockenmessung von Pulvern und Granulaten, Dispergierdruck = 2 bar).
- Kohlenstoffgehalt %C bestimmt mittels Elementaranalyse auf Kohlenstoff; Verbrennen der Probe bei über 1000 °C im O₂-Strom, Detektion und Quantifizierung des entstehenden CO₂ im IR; Gerät LECO 244
- Methanolzahl: Zur Bestimmung der Methanolzahl werden definierte Mischungen von Wasser mit Methanol hergestellt. In einem getrennten Experiment werden diese Wasser-Methanol-Mischungen mit gleichem Volumen an getrockneten Partikeln überschichtet und unter definierten Bedingungen geschüttelt (beispielweise schwaches Schütteln mit der Hand oder mit einem Taumelmischer für ca. 1 Minute). Bestimmt werden das Wasser-Alkohol-Gemisch, bei dem die Partikel eben noch nicht einsinken und das Wasser-Alkohol-Gemisch mit höherem Alkoholgehalt, bei dem die Partikel eben einsinken. Letzterer Methanolgehalt in Wasser ergibt die Methanolzahl.
- Die kinematische Viskosität wird gemessen nach DIN 53019 bei 25 °C.

In den folgenden Beispielen sind, falls jeweils nicht anders angegeben, alle Mengen- und Prozentangaben auf das Gewicht bezogen, alle Drücke 0,10 MPa (abs.) und alle Temperaturen 20°C.

### Ausführungsbeispiele

### Beispiel 1: Herstellung einer wässrigen Kieselsäuredispersion

1300 g einer teilhydrophoben pyrogenen Kieselsäure mit einem Restsilanolgehalt von 71% und einem Kohlenstoffgehalt von 0,95%, erhalten durch Umsetzung einer hydrophilen Ausgangskieselsäure mit einer spezifischen BET-Oberfläche von 200 m²/g (erhältlich unter der Bezeichnung HDK^{®} N20 bei der Wacker Chemie GmbH, München) mit Dimethyldichlorsilan gemäß EP 1433749 A1, werden portionsweise an einem Dissolver bei 650 rpm in 5200 g vollentsalztes (VE-)Wasser eingerührt. Nach vollständiger Zugabe der Kieselsäure wird noch 60 min bei 650 rpm nachdispergiert. Es wird eine hochviskose Dispersion mit 20% Feststoffgehalt und einem pH Wert von 4,2 erhalten.

### Beispiel 2: Allgemeine Vorgehensweise zur Herstellung einer Pickering Emulsion der kondensationsvernetzbaren Siliconzusammensetzungen X1 mittels Ultra-Turrax^{®}

Schritt 1: Die in Beispiel 1 beschriebene Kieselsäuredispersion wird in einem geeigneten 1000 mL Edelstahlgefäß eingewogen und mit einem Ultra-Turrax^{®} T50 bei 10000 U/min für 10 min aufgerührt. Dabei nimmt die Viskosität der Dispersion ab.

Optional wird VE-Wasser zugegeben und homogen vermischt. Die Komponenten der kondensationsvernetzbaren Siliconzusammensetzungen X1 gemäß der Beispiele 4 bis 7 werden mit einem Laborrührer vermischt und zur aufgerührten Kieselsäuredispersion gegeben und anschließend mittels Ultra-Turrax insgesamt 10 min bei 10000 U/min und Eiskühlung homogenisiert. Dabei sollte die Temperatur der Mischung nicht über 35 °C steigen. Wenn die Temperatur 35 °C übersteigt, wird das Mischen zum Kühlen unterbrochen. Es ist auch darauf zu achten, dass die gebildete Emulsion fließfähig bleibt. Sofern erforderlich wird, gegebenenfalls mehrfach, eine geringe Menge (ca. 50 mL) des Verdünnungswasser zugegeben. Es resultiert eine weiße, hochviskose Masse (Emulsion (E)).

Schritt 2: Die hochviskose Masse aus Schritt 1 wird durch Zugabe von drei gleich großen Portionen VE-Wasser auf 30% Siliconöl-Gehalt verdünnt. Nach jeder Portion VE-Wasser wird 3 Minuten bei 6000 U/min gerührt. Es entsteht eine dünnflüssige, weiße O/W-Emulsion.

### Beispiel 3: Allgemeine Vorgehensweise zur Herstellung der Hohlpartikel aus den erfindungsgemäßen Beispielen 4 bis 7 und der nicht-erfindungsgemäßen Vergleichsbeispielen V1 bis V3

Zu 250 g der polykondensationsfähigen Pickering Emulsion (E), hergestellt gemäß der allgemeinen Vorgehensweise aus Beispiel 2, werden 1,5 g p-Toluolsulfonsäure gegeben. Die Reaktionsmischung wird 24 h bei Raumtemperatur gerührt. Es resultiert eine weiße, dünnflüssige Dispersion. Die Partikel werden abfiltriert und 24 h bei 60 °C im Trockenschrank getrocknet. Man erhält ein feines, weißes Pulver.

Siliconharz S1: methoxygruppenhaltiges oligomeres Kondensationsprodukt aus Methyltrimethoxysilan mit einem Methoxygruppengehalt von ca. 30 Gewichtsprozent und der Zusammensetzung [MeSiO_{3/2}]₂₆ [MeO_{1/2}]₂₃ (Molekulargewicht laut SEC (Eluent Toluol): Mw = 2300 g/mol; Mn = 600 g/mol; Viskosität (kinematisch, DIN 51562, 25 °C) 25 mm²/s)

Siliconharz S2: ethoxygruppenhaltiges oligomeres Kondensationsprodukt aus Methyltriethoxysilan mit einem Ethoxygruppengehalt von ca. 36 Gewichtsprozent und der Zusammensetzung [MeSiO_{3/2}]₂₃ [MeO_{1/2}]₂₇ (Molekulargewicht laut SEC (Eluent Toluol): Mw = 2560 g/mol; Mn = 900 g/mol; Viskosität (kinematisch, 25 °C) 22 mm²/s.

Siliconharz S3: methoxygruppenhaltiges oligomeres Kondensationsprodukt aus Phenyltrimethoxysilan und Dimethyldimethoxysilan mit einem Molekulargewichtsmittel Mw von 1030 g/mol (Zahlenmittel Mn = 730; Polydispersität 1,4) und einer Viskosität von 140 mm²/s (25°C), das 12,3 Gew.-% Si-gebundene Methoxygruppen und 0,24 Gew.-% Si-gebundene OH-Gruppen auf der Oberfläche trägt und das durchschnittlich aus 59 Mol-% PhSiO_{3/2}-Einheiten und 41 Mol-% Me₂SiO_{2/2}-Einheiten besteht, wobei sich die Methoxygruppen auf die angegebenen Struktureinheiten verteilen.

Die Eigenschaften und Ergebnisse der Beispiele sind in Tabelle 1 zusammengefasst. Die Inhaltsstoffe der Siliconharz-Komponenten (A) und Silan-Komponenten (B) sind in Gewichtsteilen angegeben:

**Tabelle 1**

| **Beispiel** | | **4** | **5** | **6** | **7** | **V1** | **V2** | **V3** |
|---|---|---|---|---|---|---|---|---|
| **Siliconharz (A)** | **Siliconharz S1** | - | - | - | 2 | 1 | - | - |
| | **Siliconharz S2** | 2 | 2 | - | - | - | 1 | - |
| | **Siliconharz S3** | - | - | 2 | - | - | - | - |
| **Silan (B)** | **Tetraethoxysilan** | 1 | 0,5 | 1 | 1 | - | - | 1 |
| | **Methyltriethoxysilan** | - | 0,5 | - | - | - | - | - |
| **Erfindungsgemäß (ja / nein)** | | ja | ja | ja | ja | nein | nein | nein |
| **Bildung sphärische Micropartikel** | | ja | ja | ja | ja | ja | ja | nein |
| **Gestalt (gefüllt / hohl)** | | hohl | hohl | hohl | hohl | gefüllt | gefüllt | - |
| **Partikelgröße d50 (mm)** | | 2, 0 | 2,1 | 4,4 | 1, 8 | 3, 6 | 3,1 | - |
| **Schüttdichte in g/cm³** | | 0,23 | 0,21 | n.b. | n.b. | 0,3 | n.b. | - |
| **BET in m²/g** | | 186 | 185 | n.b. | n.b. | 10 | n.b. | - |

### Anwendungsbeispiele

### Beispiel 8: Anwendung in Beschichtungen

Eine erfindungsgemäße Siliconbeschichtung wurde hergestellt. Dazu wurden 2 Teile der erfindungsgemäßen Hohlpartikel aus dem Beispiel 4 mit 98 Teile ELASTOSIL^{®} RT 601 A/B (ein gießbarer, bei Raumtemperatur vulkanisierbarer, additionsvernetzender Zweikomponenten-Siliconkautschuk, zu beziehen bei Wacker Chemie AG, München, Deutschland) homogen vermischt, indem 10 min mit einem Dissolver bei 6000 upm gerührt wurde, wobei die Temperatur auf 20 °C gehalten wurde. Die resultierende Masse wurde mit einem 10 µm Rakel auf eine Glasplatte aufgebracht. Man erhält eine transparente glatte Beschichtung.

### Beispiel 9: Optische Bewertung in kosmetischer Anwendung

100 mg der erfindungsgemäßen Hohlpartikel aus dem Beispiel 5 wurden gleichmäßig auf eine kreisrunde Fläche mit 4 cm Durchmesser auf dem ungereinigten Unterarm eines Probanden verteilt. Man erhält eine trockene, gleichmäßige, optisch homogene und leicht weißliche Hautfläche. Dies ist ein Zeichen, dass vorhandenes Sebum von der Hautoberfläche vollständig adsorbiert wird.

### Beispiel 10: Anwendung als leichter Füllstoff

5 Teile der erfindungsgemäßen Hohlpartikel aus Beispiel 5 und 95 Teile ELASTOSIL^{®} LR 3003/40 A/B (einem pastösen additionsvernetzenden Zweikomponenten-Siliconkautschuk, zu beziehen bei Wacker Chemie AG, München, Deutschland) wurden mit einem Laborrührer homogen vermischt. Anschließend wurde in einer geeigneten Form ein Prüfkörper mit Durchmesser 4 cm und Höhe 0,6 cm bei 165 °C für 30 min ausgehärtet. Die Dichte des Prüfkörpers beträgt 1,01 g/mL, gemessen nach DIN EN ISO 1183-1 A.

### Vergleichsbeispiel V4:

Ein Prüfkörper wurde analog Beispiel 10 hergestellt, jedoch ohne Zugabe eines erfindungsgemäßen Hohlpartikels. Die Dichte des Vergleichsprüfkörpers beträgt 1,09 g/mL, gemessen nach DIN EN ISO 1183-1 A.

## Patentansprüche

1. Hohlpartikel P, aufgebaut aus einem hohlen Kern K und einer Hülle H, die eine Siliconharz-Zusammensetzung Z enthält, welche kondensationsvernetzte Siliconzusammensetzung X und partikulären Feststoff F enthält.

2. Hohlpartikel P nach Anspruch 1, deren mittlerer Partikeldurchmesser d50 im Bereich 0,1 - 100 µm liegt, messbar mit einem Camsizer X2 von Retsch Technology (Messprinzip:
Dynamische Bildanalyse nach ISO 13322-2, Messbereich: 0,8 µm - 8 mm, Art der Analyse: Trockenmessung von Pulvern und Granulaten, Dispergierdruck = 2 bar).

3. Hohlpartikel P nach Anspruch 1 oder 2, welche eine Sphärizität SPHT3 von mindestens 0,8 aufweisen, bestimmbar gemäß ISO 9276-6 mit einem Camsizer X2 von Retsch Technology.

4. Hohlpartikel P nach einem der vorangehenden Ansprüche, deren Hülle H einen mittleren Durchmesser von mindestens 50 nm aufweisen, jeweils ermittelt als Durchschnittswert von mindestens 5 Einzelpartikeln mittels elektronenmikroskopischen Aufnahmen wie TEM oder REM.

5. Hohlpartikel P nach einem der vorangehenden Ansprüche, bei dem das durchschnittliche Verhältnis des mittleren Durchmesser des hohlen Kern K zum mittleren Durchmesser des Hohlpartikel P größer 0,2 ist ermittelbar als Durchschnittswert von mindestens 5 Einzelpartikeln mittels elektronenmikroskopischen Aufnahmen wie TEM oder REM.

6. Hohlpartikel P nach einem der vorangehenden Ansprüche, bei dem der partikuläre Feststoff F ausgewählt wird aus Aluminium(III)-, Titan(IV)- und Silicium(IV)oxiden.

7. Hohlpartikel P nach einem der vorangehenden Ansprüche, bei dem der partikuläre Feststoffs F teilweise mit Wasser benetzbare pyrogene oder gefällten Kieselsäure oder Gemisch davon ist, die eine spezifische BET-Oberfläche von 30 bis 500 m²/g aufweist, messbar gemäß Deutscher Industrie Norm DIN 66131 und DIN 66132.

8. Hohlpartikel P nach einem der vorangehenden Ansprüche, , wobei die Methanolzahl des partikulären Feststoffs F kleiner als 70 ist, wobei zur Bestimmung der Methanolzahl definierte Mischungen von Wasser mit Methanol hergestellt werden, in einem getrennten Experiment diese Wasser-Methanol-Mischungen mit gleichem Volumen an getrockneten Partikeln überschichtet werden und unter definierten Bedingungen geschüttelt werden. Bestimmt werden das Wasser-Alkohol-Gemisch, bei dem die Partikel eben noch nicht einsinken und das Wasser-Alkohol-Gemisch mit höherem Alkoholgehalt, bei dem die Partikel eben einsinken. Letzterer Methanolgehalt in Wasser ergibt die Methanolzahl.

9. Verfahren zur Herstellung der Hohlpartikel P gemäß einem der Ansprüche 1 bis 8, bei dem in einem ersten Schritt eine Dispersion V enthaltend partikulären Feststoff F und Wasser mit kondensationsvernetzbarer Siliconzusammensetzung X1, welche bei 20°C flüssiges, alkoxygruppenhaltiges Siliconharz A und alkoxygruppenhaltiges Silan B enthält, vermischt wird, wobei eine kontinuierliche Wasser enthaltende Phase und eine diskontinuierliche Phase enthaltend kondensationsvernetzbare Siliconzusammensetzung X1 entstehen,
und in einem zweiten Schritt die Siliconzusammensetzung X1 in der diskontinuierlichen Phase zur Siliconzusammensetzung X vernetzt wird, wobei die Hohlpartikel P entstehen.

10. Verfahren nach Anspruch 9, bei dem die kondensationsvernetzbare Siliconzusammensetzung X1, jeweils bezogen auf die Gesamtmenge der Komponenten (A) und (B),
(A) 50-90 Gew.% mindestens eines Siliconharzes A,
aus Einheiten der Formeln (Ia), (Ib), (VII) und (Id)
[R¹⁷SiO_{3/2}] (Ia)
[SiO_{4/2}] (Ib)
[R¹⁷₃SiO_{1/2}] (VII)
[R¹⁷₂SiO_{2/2}] (Id)
wobei
R¹⁷ gleich oder unabhängig voneinander verschiedene einwertige, substituierte oder nicht substituierte organische Reste, die funktionelle Gruppen tragen oder nicht tragen, ein -OH oder eine Wasserstoffrest,
bedeuten,
mit den Maßgaben, dass
- in (A) mindestens 20 mol% der Formel (Ia) oder (Ib) oder einer Mischung aus beiden enthalten sind,
- in (A) höchstens 50 mol% der Formel (Ib) enthalten sind,
- Alkoxygruppen als R¹⁷ zu mindestens 5 Gew.% in (A) enthalten sind, mit der Maßgabe, dass
- (A) bei 20°C flüssig ist
(B) 10-50 Gew.-% von mindestens ein Silan B der allgemeinen Formel
R¹_{(4 -a)}Si(OR)ₐ (II),
in der
**R** einen Kohlenwasserstoffrest mit 1 bis 16 Kohlenstoffatomen dessen Kohlenstoffkette durch nicht benachbarte Gruppen -O- unterbrochen sein kann,
**R¹** gleiche oder unabhängig voneinander verschiedene einwertige Kohlenwasserstoffreste bedeutet, und
**a** die Werte 2, 3 oder 4 bedeutet, wobei mindestens 20 Gew.-% der Silane B, bezogen auf die Gesamtmasse aller Silane B, das Merkmal a = 3 oder 4 erfüllen,
enthält.

11. Verfahren nach einem oder mehreren der Ansprüche 9 bis 10, bei dem die Alkoxygruppenreste R¹⁷ des Siliconharzes A ausgewählt werden aus Methoxy-, Ethoxy-, iso-Propoxy-, n-Butoxy- und tert.-Butoxyresten.

12. Verfahren nach einem oder mehreren der Ansprüche 9 bis 11, bei dem die Reste R des Silans B ausgewählt werden aus Methyl-, Ethyl-, iso-Propyl-, und tert.-Butylresten.

13. Verfahren nach einem oder mehreren der Ansprüche 9 bis 12, bei dem der partikuläre Feststoff F ausgewählt wird aus Aluminium(III)-, Titan(IV)- und Silicium(IV)oxiden.

14. Verfahren nach einem oder mehreren der Ansprüche 9 bis 13, bei dem die mittlere Partikelgröße des partikulären Feststoffs F kleiner als 1000 nm ist, gemessen als mittlerer hydrodynamischer Äquivalentdurchmesser mittels Photonenkorrelationsspektroskopie in 173° Rückstreuung mit eine Nanosizer ZS der Fa. Malvern.

15. Verfahren nach einem oder mehreren der Ansprüche 9 bis 14, bei dem der partikuläre Feststoffs F teilweise mit Wasser benetzbare pyrogene oder gefällten Kieselsäure oder Gemisch davon ist, die eine spezifische BET-Oberfläche von 30 bis 500 m²/g aufweist, messbar gemäß Deutscher Industrie Norm DIN 66131 und DIN 66132.

16. Verfahren nach einem oder mehreren der Ansprüche 9 bis 15, bei dem der partikuläre Feststoffs F eine Methanolzahl kleiner als 70 aufweist, wobei zur Bestimmung der Methanolzahl definierte Mischungen von Wasser mit Methanol hergestellt werden, in einem getrennten Experiment diese Wasser-Methanol-Mischungen mit gleichem Volumen an getrockneten Partikeln überschichtet werden und unter definierten Bedingungen geschüttelt werden. Bestimmt werden das Wasser-Alkohol-Gemisch, bei dem die Partikel eben noch nicht einsinken und das Wasser-Alkohol-Gemisch mit höherem Alkoholgehalt, bei dem die Partikel eben einsinken. Letzterer Methanolgehalt in Wasser ergibt die Methanolzahl.

## Claims

1. A hollow particle P constructed of a hollow core K
and a shell H comprising a silicone resin composition Z that comprises condensation-crosslinked silicone composition X and particulate solid F.

2. The hollow particle P as claimed in claim 1, the median particle diameter d50 of which is in the range 0.1-100 µm, measurable using a Camsizer X2 from Retsch Technology (measurement principle: dynamic image analysis according to ISO 13322-2, measurement range: 0.8 µm to 8 mm, analysis type: dry measurement of powders and granulates, dispersion pressure = 2 bar).

3. The hollow particle P as claimed in claim 1 or 2, having a sphericity SPHT3 of at least 0.8, determinable in accordance with ISO 9276-6 using a Camsizer X2 from Retsch Technology.

4. The hollow particle P as claimed in any of the preceding claims, the shell H of which has an average diameter of at least 50 nm, in each case determined as an average value of at least 5 individual particles by means of electron microscope images, for example TEM or SEM micrographs.

5. The hollow particle P as claimed in any of the preceding claims, in which the average ratio of the average diameter of the hollow core K to the average diameter of the hollow particle P is greater than 0.2, determinable as an average value of at least 5 individual particles by means of electron microscopic images, for example TEM or SEM micrographs.

6. The hollow particle P as claimed in any of the preceding claims, in which the particulate solid F is selected from aluminum (III), titanium (IV) and silicon (IV) oxides.

7. The hollow particle P as claimed in any of the preceding claims, in which the particulate solid F is partially water-wettable fumed or precipitated silica or mixture thereof having a specific BET surface area of 30 to 500 m²/g, measurable in accordance with German industry standards DIN 66131 and DIN 66132.

8. The hollow particle P as claimed in any of the preceding claims, wherein the methanol value of the particulate solid F is less than 70, wherein defined mixtures of water with methanol are prepared for the determination of the methanol value and, in a separate experiment, these water-methanol mixtures are overlayered with the same volume of dried particles and shaken under defined conditions. The water-alcohol mixture in which the particles do not yet quite sink and the water-alcohol mixture with a higher alcohol content in which the particles just sink are determined. The latter methanol content in water gives the methanol value.

9. A process for producing the hollow particles P as claimed in any of claims 1 to 8, wherein, in a first step, a dispersion V comprising particulate solid F and water is mixed with condensation-crosslinkable silicone composition X1, which comprises alkoxy-group-containing silicone resin A that is liquid at 20°C and alkoxy-group-containing silane B, to form a continuous water-containing phase and a discontinuous phase comprising condensation-crosslinkable silicone composition X1,
and, in a second step, the silicone composition X1 undergoes crosslinking in the discontinuous phase to form the silicone composition X, resulting in the formation of the hollow particles P.

10. The process as claimed in claim 9, in which the condensation-crosslinkable silicone composition X1 comprises, respectively based on the total amount of components (A) and (B),
(A) 50-90% by weight of at least one silicone resin A,
composed of units of formulas (la), (Ib), (VII), and (Id)
[R¹⁷SiO_{3/2}] (la)
[SiO_{4/2}] (Ib)
[R¹⁷₃SiO_{1/2}] (VII)
[R¹⁷₂SiO_{2/2}] (Id)
where
R¹⁷ represent identical or independently different monovalent, substituted or unsubstituted organic radicals bearing or not bearing functional groups, or an - OH or a hydrogen radical,
with the provisos that
- at least 20 mol% of the formula (la) or (Ib) or of a mixture of the two is present in (A),
- not more than 50 mol% of the formula (Ib) is present in (A),
- alkoxy groups are present as R¹⁷ in (A) to an extent of at least 5% by weight, with the proviso that
- (A) is liquid at 20°C,
(B) 10-50% by weight of at least one silane B of the general formula
R¹₍₄₋ₐ₎Si(OR)ₐ (II),
in which
**R** is a hydrocarbon radical having 1 to 16 carbon atoms, the carbon chain of which may be interrupted by non-adjacent -O- groups,
**R¹** represents monovalent hydrocarbon radicals that are identical or independently different from one another, and
**a** represents the values 2, 3 or 4, where at least 20% by weight of silanes B, based on the total mass of all silanes B, satisfy the characteristic a = 3 or 4.

11. The process as claimed in one or more of claims 9 to 10, in which the alkoxy group radicals R¹⁷ of the silicone resin A are selected from methoxy, ethoxy, isopropoxy, n-butoxy, and tert-butoxy radicals.

12. The process as claimed in one or more of claims 9 to 11, in which the radicals R of the silane B are selected from methyl, ethyl, isopropyl, and tert-butyl radicals.

13. The process as claimed in one or more of claims 9 to 12 in which the particulate solid F is selected from aluminum (III), titanium (IV) and silicon (IV) oxides.

14. The process as claimed in one or more of claims 9 to 13, in which the median particle size of the particulate solid F is less than 1000 nm, measured as the median hydrodynamic equivalent diameter by photon correlation spectroscopy at 173° (backscattering) with a Nanosizer ZS from Malvern.

15. The process as claimed in one or more of claims 9 to 14, in which the particulate solid F is partially water-wettable fumed or precipitated silica or mixture thereof having a specific BET surface area of 30 to 500 m²/g, measurable in accordance with German industry standards DIN 66131 and DIN 66132.

16. The process as claimed in one or more of claims 9 to 15, in which the particulate solid F has a methanol value of less than 70, wherein defined mixtures of water with methanol are prepared for the determination of the methanol value and, in a separate experiment, these water-methanol mixtures are overlayered with the same volume of dried particles and shaken under defined conditions. The water-alcohol mixture in which the particles do not yet quite sink and the water-alcohol mixture with a higher alcohol content in which the particles just sink are determined. The latter methanol content in water gives the methanol value.

## Revendications

1. Particules creuses P, constituées par un noyau creux K et une enveloppe H, qui contient une composition de résine de silicone Z, qui contient une composition de silicone réticulée par condensation X et un solide particulaire F.

2. Particules creuses P selon la revendication 1, dont le diamètre moyen de particule d50 se situe dans la plage de 0,1 - 100 µm, pouvant être mesuré à l'aide d'un Camsizer X2 de la société Retsch Technology (principe de mesure : analyse dynamique d'image selon la norme ISO 13322-2, plage de mesure : 0,8 µm - 8 mm, type d'analyse : mesure à sec de poudres et de granulés, pression de dispersion = 2 bars).

3. Particules creuses P selon la revendication 1 ou 2, qui présentent une sphéricité SPHT3 d'au moins 0,8, pouvant être déterminée selon la norme ISO 9276-6 à l'aide d'un Camsizer X2 de la société Retsch Technology.

4. Particules creuses P selon l'une des revendications précédentes, dont les enveloppes H présentent un diamètre moyen d'au moins 50 nm, à chaque fois déterminé en tant que valeur moyenne d'au moins 5 particules individuelles au moyen de clichés au microscope électronique, tel qu'un MET ou un MEB.

5. Particules creuses P selon l'une des revendications précédentes, dans lesquelles le rapport moyen du diamètre moyen du noyau creux K au diamètre moyen de la particule creuse P est supérieur à 0,2, pouvant être déterminé comme valeur moyenne d'au moins 5 particules individuelles au moyen de clichés au microscope électronique, tel qu'un MET ou un MEB.

6. Particules creuses P selon l'une des revendications précédentes, dans lesquelles le solide particulaire F est choisi parmi les oxydes d'aluminium (III), de titane (IV) et de silicium (IV).

7. Particules creuses P selon l'une des revendications précédentes, dans lesquelles le solide particulaire F représente, partiellement, de la silice pyrogène ou de la silice précipitée, pouvant être mouillée par de l'eau, ou un mélange de celles-ci, et présentent une surface spécifique BET de 30 à 500 m²/g, pouvant être mesurée selon les normes DIN 66131 et DIN 66132.

8. Particules creuses P selon l'une des revendications précédentes, l'indice de méthanol du solide particulaire F étant inférieur à 70, où, pour la détermination de l'indice de méthanol, on prépare des mélanges définis d'eau et de méthanol, dans une expérience séparée, on recouvre ces mélanges eau-méthanol par un volume identique de particules séchées et on les agite dans des conditions définies, on détermine le mélange eau-alcool dans lequel les particules ne s'enfoncent tout juste pas encore et le mélange eau-alcool présentant une teneur en alcool plus élevée, dans lequel les particules s'enfoncent tout juste, la dernière teneur en méthanol dans l'eau donne l'indice de méthanol.

9. Procédé de préparation des particules creuses P selon l'une des revendications 1 à 8, dans lequel, dans une première étape, une dispersion V contenant le solide particulaire F et de l'eau est mélangée avec une composition de silicone réticulable par condensation X1, qui contient une résine de silicone A liquide à 20°C, contenant des groupes alcoxy et un silane B contenant des groupes alcoxy, une phase continue contenant de l'eau et une phase discontinue contenant une composition de silicone réticulable par condensation X1 se formant, et, dans une deuxième étape, la composition de silicone X1 dans la phase continue est réticulée en composition de silicone X, les particules creuses P se formant.

10. Procédé selon la revendication 9, dans lequel la composition de silicone X1 réticulable par condensation contient, à chaque fois par rapport à la quantité totale des composants (A) et (B),
(A) 50-90% en poids d'au moins une résine de silicone A,
constituée par des motifs des formules (Ia), (Ib), (VII) et (Id)
[R¹⁷SiO_{3/2}] (Ia)
[SiO_{4/2}] (Ib)
[R¹⁷₃SiO_{1/2}] (VII)
[R¹⁷₂SiO_{2/2}] (Id)
dans lesquelles
R¹⁷ signifie, de manière identique ou indépendamment différente, des radicaux organiques monovalents, substitués ou non substitués, qui portent ou non des groupes fonctionnels, un radical -OH ou un radical hydrogène,
étant entendu que
- dans (A) au moins 20% en mole des formules (Ia) ou (Ib) ou un mélange des deux soient contenus,
- dans (A) au plus 50% en mole de formule (Ib) soient contenus,
- des groupes alcoxy en tant que R¹⁷ soient contenus dans (A) à raison d'au moins 5% en poids, étant entendu que
- (A) soit liquide à 20°C
(B) 10-50% en poids d'au moins un silane B de formule générale
R¹₍₄₋ₐ₎Si(OR)ₐ (II),
dans laquelle
R signifie un radical hydrocarboné comprenant 1 à 16 atomes de carbone dont la chaîne carbonée peut être interrompue par des groupes -O- non adjacents,
R¹ signifie de manière identique ou indépendamment différente, des radicaux hydrocarboné monovalents et
a signifie les valeurs 2, 3 ou 4,
au moins 20 % en poids des silanes B, par rapport à la masse totale de tous les silanes B, satisfaisant à la caractéristique a = 3 ou 4.

11. Procédé selon l'une ou plusieurs des revendications 9 à 10, dans lequel les radicaux de groupe alcoxy R¹⁷ de la résine de silicone A sont choisis parmi les radicaux méthoxy, éthoxy, iso-propoxy, n-butoxy et tert-butoxy.

12. Procédé selon l'une ou plusieurs des revendications 9 à 11, dans lequel les radicaux R du silane B sont choisis parmi les radicaux méthyle, éthyle, iso-propyle et tert-butyle.

13. Procédé selon l'une ou plusieurs des revendications 9 à 12, dans lequel le solide particulaire F est choisi parmi les oxydes d'aluminium (III), de titane (IV) et de silicium (IV).

14. Procédé selon l'une ou plusieurs des revendications 9 à 13, dans lequel la grosseur moyenne de particule du solide particulaire F est inférieure à 1000 nm, mesurée en tant que diamètre équivalent hydrodynamique moyen au moyen d'une spectroscopie de corrélation de photons avec une rétrodiffusion à 173° au moyen d'un Nanosizer ZS de la société Malvern.

15. Procédé selon l'une ou plusieurs des revendications 9 à 14, dans lequel le solide particulaire F représente, partiellement, de la silice pyrogène ou de la silice précipitée, pouvant être mouillée par de l'eau, ou un mélange de celles-ci, et présente une surface spécifique BET de 30 à 500 m²/g, pouvant être mesurée selon les normes DIN 66131 et DIN 66132.

16. Procédé selon l'une ou plusieurs des revendications 9 à 15, dans lequel le solide particulaire F présente un indice de méthanol inférieur à 70, où, pour la détermination de l'indice de méthanol, on prépare des mélanges définis d'eau et de méthanol, dans une expérience séparée, on recouvre ces mélange eau-méthanol par un volume identique de particules séchées et on les agite dans des conditions définies, on détermine le mélange eau-alcool dans lequel les particules ne s'enfoncent tout juste pas encore et le mélange eau-alcool présentant une teneur en alcool plus élevée, dans lequel les particules s'enfoncent tout juste, la dernière teneur en méthanol dans l'eau donne l'indice de méthanol.
